# EUROPÄISCHE PATENTANMELDUNG

(11) **EP 1 790 356 A1**
(43) Veröffentlichungstag der Anmeldung: **30.05.2007**
(21) Anmeldenummer: 05111229.0
(22) Anmeldetag: 24.11.2005
(51) Int. Cl.: A61K 45/06, A61K 31/295, A61K 33/26, A61K 31/375

(54) **Präparat, umfassend Eisen(III)-Komplexverbindungen und redoxaktive Substanz(en)**

(71) Anmelder: VIFOR (INTERNATIONAL) AG, 9001 St. Gallen (CH)
(72) Erfinder: Ziegler, Priska, 6375 Beckenried (CH); Geisser, Peter. Dr, 9014 St. Gallen (CH)
(74) Vertreter: Gille Hrabal Struck Neidlein Prop Roos

(57) **Zusammenfassung**

Präparat, umfassend einen oder mehrere Eisen(III)-Komplexverbindungen, die bei pH 7 ein Redoxpotential von -324 mV bis -750 mV gegenüber Normalwasserstoffelektrode (NHE) aufweisen, und eine oder mehrere redoxaktive Substanzen, und dessen Verwendung,

## Beschreibung

Die vorliegenden Erfindung betrifft ein Präparat, umfassend Eisen(III)-Komplexverbindungen, die ein spezifisches Redoxpotential aufweisen, insbesondere mit Kohlehydraten oder Derivaten davon, insbesondere mit Dextrinen oder Oxidationsprodukten von Dextrinen, und eine oder mehrere redoxaktive Substanz(en), insbesondere Ascorbinsäure sowie ggf, weitere Vitamine, Spurenelemente, Mineralstoffe, Nährstoffe und/oder Cofaktoren, sowie dessen Verwendung als Medikament zur Behandlung von Eisenmangelzuständen und weiteren Erkrankungen, und die Verwendung der Eisen(III)-Komplexverbindungen zur Herstellung eines Medikaments zur Behandlung von Eisenmangel-Zuständen und weiteren Erkrankungen, wobei das Medikament gleichzeitig oder zeitnah mit redoxaktiven Substanz(en) verabreicht wird.

Eisenmangel ist der häufigste Spurenelementmangel weltweit. Ca. zwei Milliarden Menschen weltweit leiden an Eisenmangel oder Eisenmangel-Anämie (E.M, DeMaeyer, "Preventing and controlling iron deficiency anaemia through primary health care", World Health Organization, Genf, 1989, ISBN 92 4 154249 7).

Aus der WO 95/35113 ist die Verwendung von Eisen(III)-oxid als Wirkstoff zur Behandlung von Immunschwächerkrankungen, insbesondere AIDS, bekannt.

Aus der DE 1467980 sind therapeutisch verwendbare Eiseninjektionspräparate und Verfahren zu Ihrer Herstellung bekannt.

Aus der US 3076798 sind Verfahren zur Herstellung von Eisen(III)-Polymaltose-Komplexverbindungen bekannt, die zur parenteralen Verabreichung geeignet sind.

Aus der WO 04/037865 ist die Verwendung von Eisen-Kohlenhydrat-Komplexen zur Behandlung oder Prophylaxe von Eisenmangelzuständen bekannt.

Aus der WO 03/0871 64 sind Eisen-Komplexverbindungen mit hydrierten Dextrinen zur Behandlung oder Prophylaxe von Eisenmangelzuständen bekannt.

Aus der WO 02/46241 sind Eisen(III)-Pullulan-Komplexverbindungen und ihre Verwendung zur Behandlung oder Prophylaxe von Eisenmangelzuständen bekannt.

WO 99/48533 offenbart Eisen-Dextran-Verbindungen zur Behandlung von Eisenmangelanämie, die hydriertes Dextran mit einem bestimmten Molekulargewicht von ca, 1000 Dalton umfassen,

Eisensulfat ist dafür bekannt, dass es relativ häufig unangenehme dosisabhängige Nebenreaktionen, wie gastrointestinale Störungen oder eine Verfärbung der Zähne hervorruft. Eisen aus Eisensalz-Verbindungen unterliegt der passiven Diffusion freier Eisenionen. Das Eisen kann in den Kreislauf eintreten und dadurch Nebenreaktionen oder eine Eisenvergiftung hervorrufen. Dementsprechend ist auch der LD50-Wert bei weißen Mäusen mit 230 mg Eisen/kg relativ niedrig.

In Oski et al. "Effect of Iron Therapy on Behavior Performance in Nonannemic, Iron-Deficient Infants", PEDIATRICS 1983; Band 71; 877-880 ist die Verwendung von Eisen-Dextran offenbart. Die parenterale Verwendung von Eisen-Dextran ist nachteilig, weil ein Dextran-induzierter anaphylaktischer Schock auftreten kann.

Übliche orale Eisenpräparate, im allgemeinen Eisen(II)-Salze, verursachen häufig schwere gastrointestinale Nebenwirkungen, was zu einer schlechten Patienten-Compliance führt. Die orale Eisentherapie kann die Läsionen des intestinalen Gewebes durch die Katalyse der Bildung von reaktiven Sauerstoffspezies verstärken, Da freies Eisen ein starker Katalysator des Bildung von reaktiven Sauerstoffspezies ist, kann die orale Eisen(II)-Therapie, insbesondere für Patienten mit chronisch entzündlicher Darmerkrankung sogar schädlich sein. Orale Eisen(II)-Präparate werden schlecht absorbiert und führen zu hohen faecalen Eisenkonzentrationen, und ein signifikanter Anteil des faecalen Eisens ist für die katalytische Aktivität verfügbar. Wenn Eisen in Kontakt mit, ggf. bereits entzündeter intestinaler Mucosa kommt, kann es die Produktion reaktiver Sauerstoffspezies erhöhen und dadurch Gewebeschädigungen verstärken.

Eisen(III)-Polymaltose-Komplex enthält Eisen in nicht-ionischer Form, die weniger toxisch ist, Es treten bei Gabe von Verbindungen dieses Typs weniger Nebenwirkungen auf, und die Patienten-Compliance ist gegenüber Eisen(II)-sulfat verbessert (Jacobs, P., Wood, L., Bird, AR., Hematol, 2000, 5:77-83).

Verschiedenste Forschungsergebnisse haben gezeigt, dass die Menge der in der Nahrung vorhandenen Ascorbinsäure die Absorption von Eisen beeinflusst, und dass die Zugabe von Ascorbinsäure zur Nahrung die Bioverfügbarkeit des in der Nahrung enthaltenen Eisens stark verbessert (z.B. Björn-Rasmussen E. et al., Nutr. Metabol, 1974, 16, 94-100; Cook J.D. et al., Am. J. Clin. Nutr. 1977, 30, 235-241; Derman D.P. et al., Scand. J. Haematol. 1980, 25, 193, Gillooly C. et al., Scand. J. Haematol. 1982, 29, 18-24; Hallberg L., Ann. Rev. Nutr. 1981, 1, 123-147; Hallberg, L. et al., Am. J. Clin. Nutr. 1984, 39, 577; Morch, T.A. et al., Am. J. Clin. Nutr. 1982, 36, 219-223; Sayers M.H., Br. J. Haematol. 1973, 24, 209-218; Sayers M.H. et al., Br. J. Nutr. 1974, 31 , 367-375 ; Sayers M.H et al., Br. J. Haematol. 1972, 28, 483-495). Oft wird dabei angenommen, dass die Reduktion des schlechtlöslichen, dreiwertigen Eisens zum gutlöslichen zweiwertigen Eisen eine entscheidende Rolle spielt.

Aus dieser Überlegung heraus wurden spezielle Eisenpräparate entwickelt, welche Ascorbinsäure enthalten, und diese sind heute im Markt gut vertreten. Es handelt sich bei diesen Präparaten um Kombinationen von Eisen(II)-salzen, vorwiegend Eisen (II)-sulfat, mit Ascorbinsäure. Die Ascorbinsäure dient bei diesen Präparaten dazu, die Oxidation von Eisen (II)- zu Eisen(III) im Präparat zu verhindern.

Weiter ist bekannt, dass Eisen(II)-Salze mit Ascorbinsäure einen farbigen Komplex bilden, und dass Ascorbinsäure mit Eisen(III)-chlorid bei saurem pH einen löslichen Chelatkomplex bildet, jedoch nicht mit Eisen(III)-Niederschlägen bei alkalischem pH. Der lösliche Eisen(III)-chelatkomplex ist stabil und hält Eisen in löslicher Form auch bei anschließender Alkalinisierung der Lösung (Conrad, M.E, et al., Gastroenterolgy 1968, 55, 35-45).

Durch Mössbauer- und UV/VIS-Spektroskopie unter sauerstofffreien Bedingungen konnte gezeigt werden, dass Ascorbinsäure im pH-Bereich 6-7 mit Fe²⁺ keine Komplexe bildet, jedoch mit Fe³⁺ (Hamed, M.Y. et al., Inorg. Chim. Acta 1988, 152, 227-231). Fe²⁺ bildet mit Ascorbinsäure keine Komplexe, und daher werden im alkalischen Bereich Ausfällungen beobachtet, im Gegensatz zum System Fe³⁺ mit Ascorbinsäure, das bei neutralem und alkalischem pH als Fe(III)-Komplexlösung vorliegt (Gorman, J.E, et al., J. of Food Science 1983, 48, 1217-1225). Fe³⁺ bildet mit Ascorbinsäure bei pH 6,5 einen roten, wasserlöslichen 1:1 Komplex (Hamed, M.Y. et al., Inorg. Chim Acta 1988, 12, 227-231).

Eisen (III)-Komplexe lassen sich in folgende zwei Gruppen einteilen:
- solche, die unter physiologischen Bedingungen (pH7) mit NADP(H) zu Eisen (II) reduzierbar sind
- solche, die unter diesen Bedingungen nicht reduzierbar sind.

Das entscheidende Redoxpotential liegt hierfür bei -324 mV. Dies ist das Redoxpotential von NAD(P)H/NADP⁺ bei pH 7.

Bekannt ist, dass das Redoxpotential für die Reaktion von Ascorbinsäure zu Dehydroascorbinsäure bei pH 7 bei -66 mV liegt (Borsook, H. et al., Proc. N.A.S. 1933, 875-878). Das bedeutet, dass Eisen(III)-Komplexe mit einem Redoxpotential von < -66 mV bei pH 7 nicht durch Ascorbinsäure reduziert werden können, Eisen(III)-Polymaltose-Komplex weist bei pH 7 ein Redoxpotential von -332 mV auf (Crichton, R.R., Danielson, B.G., Geisser, P. Iron Therapy with special emphasis on intravenous administration, 2. Auflage, UNI-MED Verlag AG, Bremen, 2005, S, 44, Fig. 6.4).

In der gesamten vorliegenden Patentanmeldung sind alle Redoxpotentiale stets gegenüber einer Normalwasserstoffelektrode (NHE) gemessen und angegeben.

Bereits ältere Studien haben die Reduktion von Eisen³⁺ zu Eisen²⁺ unter gastro-intestinalen Bedingungen angezweifelt (Gorman, J.E. et al., J. of Food Science 1983, 48, 121 7-1225), Neuere Studien zeigen, dass bei stark saurem pH eine Reduktion stattfindet, dass aber die Bildung eines Eisen(III)-Ascorbinsäure-Komplexes die schnellere Reaktion ist und auch bei höheren pH-Werten stattfinden kann (Dorey, C. et al., Iron Club Meeting 1988; Xu, J. et al, Inorg. Chem 1990, 29, 4180-4184).

Bereits 1968 wurde gezeigt, dass FeCl₂ und FeCl₃ in Kombination mit Ascorbinsäure weit bessere Absorptionsresultate zeigen als ohne (Conrad, M,E, at al., Gastroenterologie 1968, 55, 35-45), Konkret wurde in dieser Studie gezeigt, dass Fe(III) aus FeCl₃ etwa gleich gut resorbiert wird wie Fe(II) aus FeCl₂, dass Ascorbinsäure einen Absorptions-Steigerungseffekt für Fe(II) und Fe(III) bewirkt, dass von den untersuchten Präparaten der Fe(III)-Ascorbinsäure-Komplex die beste Absorption aufweist, und dass das entsprechende Präparat nicht nur bei anämischen, sondern auch bei nicht anämischen Ratten eine höhere Absorption aufweist als die anderen untersuchten Präparate.

Derman ((Derman, D.P. et al., Scand. J. Haematol. 1980, 25, 193-201) konnte zeigen, dass die Absorption von 3 mg Fe als Ferritin bzw. Eisen(III)-hydroxid aus eines Mais-Porridge-Mahlzeit durch die Zugabe von 100 mg Ascorbinsäure von je 0,4 % auf 12,1 bzw. 10,5 % verbessert wird. Der Anteil von dialysierbarem Eisen konnte durch Komplexbildung mit Ascorbinsäure bei pH 3,0 und 7,0 enorm verbessert werden.

Es ist weiterhin bekannt, dass Säure die Absorption von Eisen(II)-Salzen oder Hämoglobin-Eisen nicht erhöht, jedoch die Absorption von Eisen aus Eisen(III)-Salzen und der Nahrung erhöhen könnte (Conrad, M,E, et al., Gastroenterology 1968, 55, 35-45), Ionisches Eisen (III) wird von der intestinalen Mucosa nicht absorbiert, und daher müssen gastrointestinale Sekrete ionisches Eisen(III) zunächst zu ionischem Eisen(II) reduzieren oder es chelatisieren, um das Eisen zu lösen und seine Absorption zu erhöhen.

Eine weitere Studie von Naito (Naito, Y, et al., Digestion 1995, 56, 472-478) hat gezeigt, dass Eisen(II)-ionen in Kombination mit Ascorbinsäure locale Ulcerationen im Magen-Darm-Trakt auslösen, und dass die durch Sauerstoffradikale vermittelte Lipidperoxidation eine entscheidende Rolle bei der Pathogenese von gastrischen Ulcerationen spielt, die durch Eisen(II) in Kombination mit Ascorbinsäure ausgelöst werden.

Früher wurde angenommen, dass hohe Konzentrationen von Ascorbinsäure die Lipidperoxidation inhibieren, vermutlich aufgrund direkter Antioxidations-Eigenschaften (Bucher, J.R., et al, Fund. Appl. Tox. 1983, 3, 222-226), aber auch durch Halten von Eisen ausschließlich in der reduzierten Form (Baughler, J.M. et al., J. Biol. Chem. 261, 10282-10289).

Eisen (II) reagiert aber mit Sauerstoff zu Eisen (III) und freien OH-Radikalen. Die Bildung dieser Radikale führt zu intensiven Nebenwirkungen und ist unerwünscht. Eine Studie von Fodor hat in neuerer Zeit gezeigt (Fodor, I. et al., Biochim. Biophys. Acta 961 (1988), 96-102), dass die Kombination von Eisen (II) mit Ascorbinsäure zu signifikant mehr Ulcerationen im Magen-Darm-Trakt führt als Eisen(II) allein. Weiter hat diese Studie gezeigt, dass Ascorbinsäure in Kombination mit Eisen(II) ein Promoter der Lipidperoxidation ist und kein Inhibitor wie früher angenommen, da die Kombination von Eisen(II) mit Ascorbinsäure von allen untersuchten Kombinationen die intensivste Lipidperoxidation induzierte. Die Lipidperoxidation ist ihrerseits verantwortlich für Schädigungen biologischer Membranen und damit für Ulcerationen.

Auch weitere Studien zeigen immer wieder die Toxizität der Kombination von Eisen(II) und Ascorbinsäure auf (Higson, F.K., et al., Free Rad. Res. Comms. 1988, 5, 107-115; Uchida, K. et al., Agric. Biol. Chem. 1989, 53, 3285-3292). Die Schäden die durch diese toxischen Effekte auf Zellebene entstehen, geben dann Anlass zu Nebenwirkungen und einer schlechten Patienten-Compliance.

Oxidativer Stress, insbesondere die Lipidperoxidation, wird z.B. mit einem erhöhten Risiko, an Herzinfarkt, Krebs und Atherosklerose zu erkranken, in Verbindung gebracht. Die oxidative Modifizierung von Low-Density Lipoprotein (LDL) wird für die Atherogenese verantwortlich gemacht (s. in Tuomainen et al., Nutrition Research, Vol 19, No.8, pp. 1121-1132, 1999 angegebene Referenzen).

Die Erfinder stellten sich daher die Aufgabe, gut verträgliche Eisen(III)- Präparate in Kombination mit einer oder mehreren redoxaktiven Substanz(en) zu finden, die geeignet sind, Eisenmangel-Zustände zu behandeln, und die eine verbesserte Bioverfügbarkeit des Eisens gewährleisten, ohne die oben beschriebenen nachteiligen Auswirkungen der bekannten Eisen(II)-Ascorbinsäure-Kombinationsräparate wie Bildung von Ulcerationen im Magen-Darm-Trakt und oxidativer Stress durch Lipidperoxidation zu zeigen.

Aufgabe der Erfindung war daher, eine Kombination von Eisen(III) und einer oder mehreren redoxaktiven Subsanz(en) bereitzustellen, in der das Eisen(III) nicht durch die redoxaktive(n) Substanz(en), insbesondere Ascorbinsäure, zu Eisen(II) reduziert wird und somit keinen oxidativen Stress verursacht. Die optimale Absorptionsmöglichkeit für Eisen durch Bildung von Eisen(III)-Komplexen mit der/den redoxaktiven Substanz(en) sollte dagegen ausgenützt werden.

Diese Aufgabe wird gelöst durch das erfindungsgemäße Präparat, das Eisen(III)-Komplexverbindungen mit einem spezifischen Redoxpotential, insbesondere solche mit Kohlenhydraten oder Derivaten davon, und eine oder mehrere redoxaktive Substanz(en), insbesondere Ascorbinsäure, umfasst.

Eisen(III)-Komplexverbindungen mit Kohlehydraten, insbesondere mit Polymaltose (Maltodextrin) sind besonders verträglich und besitzen eine hohe Patienten-Compliance, Unter der Behandlung mit den Eisen(III)-Komplexen tritt kein oxidativer Stress auf.

Untersuchungen der Erfinder haben gezeigt, dass Eisen(III)-Polymaltosekomplex, welcher bei pH 7 ein Reduktionspotential von -332 mV aufweist, bei pH 3, 5,5 und 8 in gepufferter Lösung (Puffersysteme pH 3.0: 10⁻³ mol/l HCl; pH 5,5 und pH 8,0: 0,1 mol/l NH₄Cl/NH₃; s. Geisser, P., Arzneim.-Forsch./Drug Res. 1990, 40 (II), 7, 754-760) unter Sauerstoffausschluss nicht mit Ascorbinsäure zu Dehydroascorbinsäure reagiert.

Eisen(III)-Polymaltose-Komplex-Verbindungen führen zwar nur zu einer langsamen Erhöhung des Ferritinspiegels, werden aber effizienter für die Hämoglobin-Synthese verwendet (T.-P. Tuomainen et al., aaO., p. 1 127).

Unter Eisenmangel-Zustand gemäß der Erfindung wird ein Zustand verstanden, bei dem Hämoglobin, Eisen und Ferritin im Plasma vermindert sind und Transferrin erhöht ist, was zu einer erniedrigten Transferrin-Sättigung führt.

Der erfindungsgemäß zu behandelnde Zustand umfasst Elsenmangelanämie und Eisenmangel ohne Anämie. Die Einteilung kann beispielsweise durch den Hämoglobinwert und den Wert für die Transferrinsättigung (%) erfolgen. Referenzwerte für Hämoglobin, bestimmt durch Durchflusszytometrie oder die photometrische Cyanhämoglobinmethode, und Referenzwerte für Eisen, Ferritin und Transferrin sind beispielsweise gelistet in der Referenzdatenbank der Charité, Institut für Laboratoriumsmedizin und Pathobiochemie (http://www.charite.de/ilp/routine/parameter.html) und in Thomas, L. Labor und Diagnose, TH Book Verlagsgesellschaft, Frankfurt/Main 1998, Die Transferrinsättigung ist bei Patienten ohne Eisenmangel in der Regel > 16 %. Der Ferritin-Wert beträgt bei Patienten ohne Eisenmangel in der Regel mindestens 30 µg/l, und der Hämoglobinwert mindestens 130 g/l.

Laut M. Wick, W. Pinggera, P. Lehmann, Eisenstoffwechsel - Diagnostik und Therapien der Anämien, 4., erw. Aufl, Springer Verlag Wien 1998 lassen sich alle Formen des Eisenmangels klinisch-chemisch nachweisen. Dabei geht im allgemeinen eine erniedrigte Ferritin-Konzentration mit kompensatorisch erhöhtem Transferrin und niedriger Transferrinsättigung einher.

Weiter ist das erfindungsgemäße Präparat zur Verbesserung der Immunabwehr und zur Verbesserung der Hirnleistung einsetzbar,

Verbesserung der Immunabwehr im Sinne der Erfindung bedeutet eine signifikante Verbesserung der Immunantworten, wie sie sich beispielsweise in einer signifikanten Verbesserung der Lymphozytenantwort gegenüber Phytohämagglutinin (PHA) unter Verwendung der MTT-Methode, in einer Verbesserung im Nitroblau-Tetrazoliumtest (MBT) unter Verwendung von Neutrophilen, in einer Verbesserung der bakteriziden Kapazität von Neutrophilen (PCA) gemessen durch das turbidimetrische Verfahren, in einer Verbesserung der monoklonalen Antikörper, beispielsweise CD3, CD4, CD8 und CD56, ausgezählt beispielsweise mit einem BD-Fließzytometer mit einfacher Färbemethode und/oder in der Antikörperantwort gegenüber Masern, H-Influenza und Tetanus zeigt. Die erfindungsgemäße Verwendung verläuft in den letztgenannten Fällen insbesondere mittels Verbesserung der Neutrophilen-Niveaus, der Antikörperniveaus und/oder der Lymphozytenfunktion, bestimmt beispielsweise durch die Lymphozytenreaktion gegenüber Phytohämagglutinin.

Eine Verbesserung der Hirnleistung im Sinne der Erfindung schließt insbesondere eine Verbesserung der kognitiven Funktionen und des emotionalen Verhaltens ein und drückt sich beispielsweise in einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-I, YV-Test; Jugendversion) aus.

Das erfindungsgemäße Präparat ist auch zur Behandlung des "Restless Legs-Syndroms" einsetzbar (RLS, auch als Ekbom's Syndrom bekannt). Es handelt sich um eine Erkrankung, bei der die Patienten nicht in der Lage sind, sitzen zu bleiben oder gar still zu stehen. Die Patienten leiden weiter wegen des unwiderstehlichen Bewegungsdrangs unter ausgeprägter Schlaflosigkeit, Sobald der Patient sich bewegt, verschwinden die Symptome, kehren aber sofort zurück, wenn die Bewegung aufhört, Wenn Patienten zum Liegen gezwungen werden, beobachtet man unfreiwillige Beinbewegungen. Für weitere Erläuterungen dieser Indikation wird auf WO 2004/083693 verwiesen.

Das erfindungsgemäße Präparat ist weiter zur Behandlung von Eisenmangelzuständen bei Patienten mit chronisch entzündlichen Darmerkrankungen, insbesondere Morbus Crohn und Colitis ulcerosa, geeignet.

Auch zur Behandlung oder Vorbeugung von Eisenmangelzuständen bei Schwangeren ist das erfindungsgemäße Präparat besonders geeignet, insbesondere, wenn noch weitere pharmakologisch wirksame Bestandteile in Form von Vitaminen abgesehen von Ascorbinsäure, Mineralstoffen, Spurenelementen, Nährstoffen und/oder Spurenelementen enthalten sind, wie im folgenden beschrieben.

Erfindungsgemäß anwendbare Eisen(III)-Komplexverbindungen sind solche mit einem Redoxpotential bei pH 7 von -324 mV bis -750 mV, bevorzugt -330 mV bis -530 mV, besonders bevorzugt -332 mV bis -475 mV. Diese Bedingungen werden insbesondere von bestimmten Eisen(III)-Polymaltosekomplexen, Eisen(III)-Dextrin-Komplexen, Eisen(III)-Dextran-Komplexen und Eisen(III)-Sucrose-Komplexen sowie dem Eisen(III)-Transferrin-Komplex erfüllt. Von diesen sind Eisen(III)-Polymaltose-Komplexe mit dem genannten Redoxpotential besonders bevorzugt. Es sind aber auch andere Eisen(III)-Komplexverbindungen geeignet, solange sie ein Redoxpotential im angegebenen Bereich aufweisen.

Erfindungsgemäß anwendbare Eisen(III)-Komplexverbindungen sind insbesondere solche mit Kohlenhydraten. Sie schließen bevorzugt diejenigen ein, worin Kohlenhydrate aus der Gruppe ausgewählt werden, die aus Dextranen und Derivaten davon, Dextrinen und Derivaten davon sowie Pullulan, Oligomeren und/oder Derivaten davon besteht. Die genannten Derivate umfassen insbesondere die hydrierten Derivate. Besonders bevorzugt sind Eisen(III)-Komplexverbindungen mit Dextrinen oder Oxidationsprodukten davon. Beispiele der Herstellung der erfindungsgemäßen Eisen(III)-Komplexverbindungen finden sich beispielsweise in den eingangs erwähnten Patentschriften DE 14679800, WO 04037865 A1, US 3076798, WO 03/087164 sowie WO 02/46241, deren Offenbarungsgehalt insbesondere hinsichtlich der Herstellverfahren hier vollumfänglich eingeschlossen sein soll, Der Begriff der erfindungsgemäß bevorzugt verwendeten "Dextrine" ist eine Sammelbezeichnung für verschiedene niedere und höhere Polymere aus D-Glucose-Einheiten, die bei unvollständiger Hydrolyse von Stärke entstehen. Dextrine können ferner durch Polymerisation von Zuckern hergestellt werden (z,B, WO 02083739 A2, US 20030044513 A1, US 3766165). Zu den Dextrinen gehören die Maltodextrine bzw, Polymaltosen, die durch enzymatische Spaltung von zum Beispiel Mais- oder Kartoffelstärke mit alpha-Amylase hergestellt werden und die durch den Hydrolysegrad ausgedrückt durch den DE-Wert (Dextrose-Äquivalent) charakterisiert werden. Polymaltose kann erfindungsgemäß auch durch saure Hydrolyse von Stärken, insbesondere von Dextrinen erhalten werden. Die Herstellung der erfindungsgemäß anwendbaren Eisen(III)-Komplexverbindungen erfolgt im allgemeinen durch Umsetzung von Eisen(II)- oder (III)-salzen, insbesondere Eisen(III)-chlorid, mit den Dextrinen, insbesondere Polymaltose, oder Oxidationsprodukten der Dextrine in wässriger alkalischer Lösung (pH > 7) und anschließender Aufarbeitung. Die Herstellung gelingt auch im schwach sauren pH-Bereich. Bevorzugt sind jedoch alkalische pH-Werte von beispielsweise >10.

Das Anheben des pH-Wertes erfolgt bevorzugt langsam bzw. allmählich, was beispielsweise dadurch erfolgen kann, dass zunächst eine schwache Base zugesetzt wird, beispielsweise bis zu einem pH von etwa 3; anschließend kann dann mit einer stärkeren Base weiter neutralisiert werden, Als schwache Base kommen beispielsweise Alkali- oder Erdalkalicarbonate, -bicarbonate, wie Natrium- und Kaliumcarbonat oder -bicarbonat oder Ammoniak infrage. Starke Basen sind beispielsweise Alkali- oder Erdalkalihydroxide, wie Natrium-, Kalium-, Calcium-oder Magnesiumhydroxid.

Die Umsetzung kann durch Erwärmen begünstigt werden. Beispielsweise können Temperaturen in der Größenordnung von 15°C bis zur Siedetemperatur angewendet werden. Es ist bevorzugt, die Temperatur allmählich zu steigern. So kann beispielsweise zunächst auf etwa 15 bis 70°C erwärmt und allmählich bis zum Sieden gesteigert werden.

Die Reaktionszeiten liegen beispielsweise in der Größenordnung von 15 Minuten bis zu mehreren Stunden, z.B. 20 Minuten bis 4 Stunden, beispielsweise bei 25 bis 70 Minuten, z. B. 30 bis 60 Minuten.

Nach erfolgter Umsetzung kann die erhaltene Lösung beispielsweise auf Raumtemperatur abgekühlt und gegebenenfalls verdünnt und gegebenenfalls filtriert werden. Nach dem Abkühlen kann der pH-Wert durch Zugabe von Säure oder Base auf den Neutralpunkt oder leicht darunter, beispielsweise auf Werte von 5 bis 7 eingestellt werden. Als Basen können beispielsweise die vorstehend zur Umsetzung genannten verwendet werden. Säuren schließen beispielsweise Salzsäure und Schwefelsäure ein. Die erhaltenen Lösungen werden gereinigt und können direkt zur Herstellung von Arzneimitteln verwendet werden. Es ist aber auch möglich, die Eisen(III)-Komplexe aus der Lösung zu isolieren, beispielsweise durch Ausfällen mit einem Alkohol, wie einem Alkanol, beispielsweise Ethanol. Die Isolierung kann auch durch Sprühtrocknung erfolgen. Die Reinigung kann in üblicher Weise erfolgen, insbesondere zur Entfernung von Salzen, Dies kann z. B. durch Umkehrosmose erfolgen, wobei eine derartige Umkehrosmose z. B. vor der Sprühtrocknung oder vor dem direkten Einsatz in Arzneimitteln durchgeführt werden kann.

Die erhaltenen Eisen(III)-Komplexe weisen beispielsweise einen Eisengehalt von 10 bis 40 % Gew./Gew., insbesondere 20 bis 35% Gew./Gew. auf. Sie sind im allgemeinen gut wasserlöslich, Man kann daraus neutrale wässrige Lösungen mit beispielsweise 1 % Gew./Vol. bis 20 % Gew./Vol. Eisengehalt herstellen. Diese Lösungen lassen sich thermisch sterilisieren.

Bezüglich der Herstellung von Eisen(III)-Polymaltose-Komplexverbindungen kann auch auf die US 3076798 verwiesen werden.

In einer bevorzugten Ausführungsform der Erfindung wird eine Eisen(III)-hydroxid-Polymaltose-Komplexverbindung verwendet. Bevorzugt besitzt diese die Eisen(III)-Polymaltose-Komplexverbindung ein Molekulargewicht im Bereich von 20000 bis 500000, in einer bevorzugten Ausführungsform 30000 bis 80000 Dalton (bestimmt mittels Gelpermeationschromatographie, beispielsweise wie von Geisser et al, in Arzneim. Forsch/Drug Res. 42(11), 12,1439-1452 (1992), Absatz 2.2. 5. beschrieben). Eine besonders bevorzugte Eisen(III)-hydroxid-Polymaltose-Komplexverbindung ist das im Handel erhältlich Maltofer® der Firma Vifor (International) AG, Schweiz. In einer weiteren bevorzugten Ausführungsform wird eine Eisen(III)-Komplexverbindung mit einem Oxidationsprodukt von einem oder mehreren Maltodextrinen verwendet. Diese ist beispielweise erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 37 und beim Einsatz eines Gemisches aus mehreren Maltodextrinen das Dextrose-Äquivalent des Gemisches bei 5 bis 37 und das Dextrose Äquivalent der am Gemisch beteiligten einzelnen Maltodextrine bei 2 bis 40 liegt. Das gewichtsmittlere Molekulargewicht Mw der so erhaltenen Komplexe beträgt beispielsweise 30 kDa bis 500 kDa, bevorzugt 80 bis 350 kDa, besonders bevorzugt bis zu 300 kDa (bestimmt mittels Gelpermeationschromatographie, beispielsweise wie von Geisser et al. In Arzneim. Forsch/Drug Res. 42(11), 1 2,1439-1452 (1992), Absatz 2, 2, 5, beschrieben), Diesbezüglich kann beispielsweise auf die WO 2004037865 A1 verwiesen werden, deren Offenbarungsgehalt vollumfänglich in vorliegender Anmeldung eingeschlossen sein soll.

Bezüglich der Herstellung von Eisen-Komplexverbindungen mit hydrierten Dextrinen kann auf die WO 03/087164 verwiesen werden.

Bezüglich der Herstellung von Eisen(III)-Pullulan-Komplexverbindungen kann auf die WO 02/46241 verwiesen werden.

Als redoxaktive Substanz(en) ist/sind erfindungsgemäß Ascorbinsäure, Vitamin E, Cystein, physiologisch verträgliche Phenole/Polyphenole und Gluthathion verwendbar. Als physiologisch verträgliche Phenole/Polyphenole kommen z.B. Quercetin, Rutin, Flavone, andere Flavonoide (z.B. Campherole) und Hydrochinone in Frage, insbesondere Quercetine, sowie Derivate der genannten Verbindungen. Besonders bevorzugt ist Ascorbinsäure. Es können eine oder mehrere dieser redoxaktiven Substanzen eingesetzt werden, besonders bevorzugt sind die Kombination von Vitamin E mit Ascorbinsäure und Ascorbinsäure allein.

Im erfindungsgemäßen Präparat liegen Eisen(III)-Komplexverbindung und redoxaktive Substanz(en), insbesondere Ascorbinsäure, bevorzugt im Gewichtsverhältnis 1:0,05 bis 1 :20 vor, bevorzugt 1 : 0,3 bis 1 : 2, besonders bevorzugt 1 : 0,4 bis 1 : 1,8, am bevorzugtesten 1 : 1,5 (bezogen auf die Eisen(III)-Komplexverbindung, nicht auf Eisen(III)),

Das erfindungsgemäße Präparat kann ggf, weitere pharmakologisch wirksame Bestandteile enthalten, die aus der aus Vitaminen abgesehen von Ascorbinsäure, Spurenelementen, Mineralstoffen, Nährstoffen und Cofaktoren bestehenden Gruppe ausgewählt werden. Der/die weiteren pharmakologisch wirksamen Bestandteile sind bevorzugt die Vitamine β-Carotin, Thiamin (Vitamin B₁), Riboflavin (Vitamin B₂), Pyridoxin (Vitamin B₆), Cyanocobalamin (Vitamin B₁₂), Cholecalciferol (Vitamin D₃), α-Tocopherol (Vitamin E), Biotin (Vitamin H), die Cofaktoren Pantothensäure, Nicotinamid, Folsäure, die Spurenelemente/Mineralien Kupfer, Mangan, Zink, Calcium, Phosphor und/oder Magnesium und die Nährstoffe Aminosäuren, Oligopeptide, Kohlenhydrate und Fette, gegebenenfalls in Form physiologisch verträglicher Salze. Als physiologisch verträgliche Salze kommen alle üblichen physiologisch verträglichen Salze in Frage, bevorzugt Salze anorganischer Säuren oder Basen wie Hydrochloride, Sulfate, Chloride, Phosphate, Hydrogenphosphate, Dihydrogenphosphate, Hydroxide oder Salze organischer Säuren wie z.B. Acetate, Fumarate, Maleate, Citrate usw. Die weiteren pharmakologische wirksamen Bestandteile können auch als Hydrate oder Solvate vorliegen. Phosphor wird bevorzugt in Form von Phosphaten oder Hydrogenphosphaten zugesetzt.

Da Ascorbinsäure bei neutralem pH mit Luftsauerstoff zu Dehydroascorbinsäure oxidiert wird, sind Präparate in Form von üblichen, der Luft ausgesetzten Lösungen erfindungsgemäß wenig bis gar nicht geeignet.

Präparate, die über einen längeren Zeitraum stabil sind, wie Tabletten (Kau-, Film-, Brausetabletten), Brausegranulate, Pulvermischungen, Kapseln, Sachets oder aber Kits, in denen der Eisen(III)-Komplex und ggf. weitere Bestandteile in Lösung, z.B. in Portions-Vials oder -flaschen vorliegt und die Redoxaktiven Substanz(en), insbesondere Ascorbinsäure, bevorzugt in Pulver- oder Granulatform, unmittelbar vor der Einnahme zugesetzt wird, sind jedoch gut geeignet. Als Lösungsmittel für die letztgenannten Lösungen dient i.a. Wasser, es sind aber auch übliche Sirupgrundlagen oder Säfte geeignet. Besonders bevorzugt sind erfindungsgemäß Single-Dose-Container (Monodosenbehälter), d.h. Gefäße, bevorzugt aus Glas, deren Deckel als Behälter für die redoxaktiven Substanzen ausgestaltet ist, die dann kurz vor der Einnahme durch Durchdrücken des Deckelbodens in das Gefäß eingebracht und mit dessen Inhalt gemischt wird. Derartige Single-Dose-Container sind bekannt und auf dem Markt erhältlich.

Weiter ist es erfindungsgemäß vorgesehen, die Eisen(III)-Komplexverbindung gleichzeitig oder zeitnah mit der/den redoxativen Substanzen, insbesondere Ascorbinsäure einzunehmen, wobei die redoxaktive(n) Substanz(en) bevorzugt in Form einer Lösung, besonders bevorzugt als Fruchtsaft, insbesondere Orangensaft eingenommen wird.

Zeitnah bedeutet dabei, dass beide Komponenten im Abstand von höchstens 2 Stunden gegeben werden, bevorzugt höchstens 30 Minuten.

Dabei werden bevorzugt 40 mg bis 120 mg, bevorzugter 60 mg bis 100 mg Eisen(III) (berechnet als Eisen (III), nicht als Eisen(III)-Komplex) als Tablette, Kapsel, Tropfen, Saft, Dragees oder andere orale galenische Zubereitung mit 100 ml Orangensaft, entsprechend einem Ascorbinsäure-Gehalt von ca. 150 mg, eingenommen, Besonders bevorzugt sind Tabletten, die 60 mg oder 100 mg Eisen(III) enthalten, Ggf, können weitere pharmakologisch wirksame Bestandteile wie vorstehend erläutert entweder in der Zubereitung des Eisen(III)-Komplexes oder in der Lösung der redoxaktiven Substanz(en) oder in beiden enthalten sein.

Die Eisen(III)-hydroxid-Komplex-Verbindungen und die redoxative(n) Substanz(en) sowie ggf. weitere Bestandteile können mit üblichen pharmazeutischen Träger- bzw, Hilfsstoffen in die geeignete Darreichungsform gebracht werden. Dazu können übliche Bindemittel bzw. Gleitmittel, Verdünnungsmittel, Sprengmittel, Füllstoffe etc. verwendet werden, Tabletten können mit üblichen Filmbildnern beschichtet werden. Weiter können Aroma- , Geschmacks- und Farbstoffe zugesetzt werden, falls gewünscht.

Die erfindungsgemäß verwendeten Eisen(III)-hydroxid-Komplexverbindungen werden oral verabreicht. Die tägliche Dosis beträgt beispielsweise zwischen 10 und 500 mg Eisen(III)/Tag der Anwendung. Patienten mit Eisenmangel oder Eisenmangel-Anämie nehmen z.B. 2 bis 3 mal täglich je 100 mg Eisen(III) ein, und Schwangere 1 bis 2 mal täglich 60 mg Eisen(III) (jeweils berechnet als Eisen(III), nicht als Komplex).

Die tägliche Dosis redoxaktive Verbindung, insbesondere Ascorbinsäure, beträgt beispielsweise 50 bis 300 mg täglich, bevorzugt ca. 150 mg entsprechend in etwa einem Glas Orangensaft.

Die Verabreichung kann bedenkenlos über einen Zeitraum von mehreren Monaten bis zur Verbesserung des Eisenstatus, reflektiert durch den Hämoglobin-Wert, die Transferrin-Sättigung und den Ferritin-Wert der Patienten, oder zur gewünschten Verbesserung der Hirnleistung oder Immunantwort oder der Besserung der Symptome des Restless Legs-Syndrom erfolgen.

Das erfindungsgemäße Präparat kann von Kindern, Jugendlichen und Erwachsenen eingenommen werden.

Die erfindungsgemäße Verwendung verläuft insbesondere mittels Verbesserung der Eisen-, Hämoglobin-, Ferritin- und Transferrinwerte. Einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder einer Verbesserung der Neutrophilen-Niveaus, der Antikörperniveaus und/oder der Lymphozytenfunktion,

Die Erfindung wird in ihrer Wirkungsweise durch die folgenden Beispiele erläutert und belegt,

### BEISPIELE

### Beispiel 1

Es wurden auf übliche Weise beschichtete Filmtabletten hergestellt, die pro Stück folgende Bestandteile enthielten:

| | |
|---|---|
| β-Carotin | 7,2 mg |
| Vitamin B1 (als Thiamin-Nitrat) | 2,0 mg |
| Vitamin B2 | 1,8 mg |
| Vitamin B6 (als Pyridoxin Hydrochlorid) | 2,7 mg |
| Vitamin B12 | 0,0026 mg |
| Ascorbinsäure | 95 mg |
| Vitamin D3 | 10 µg |
| Vitamin E | 12 mg |
| Biotin | 0,1 mg |
| Calciumpantothenat | 7,6 mg |
| Nicotinamid | 20 mg |
| Folsäure | 0,8 mg |
| Kupfersulfat, wasserfrei | 5 mg |
| Manganchlorid-Tetrahydrat | 11 mg |
| Zinksulfat-Monohydrat | 52 mg |
| Calciumhydrogenphosphat, wasserfrei | 439 mg |
| Magnesiumoxid | 166 mg |
| Eisen(III)-Polymaltose-Komplex Fe(III)) | 226 mg (60 mg |
| Croscarmellose-Natrium | 41 mg |
| Kolloidales wasserfreies Siliziumoxid | 7 mg |
| Magnesiumstearat | 6 mg |
| Mikrokristalline Cellulose | 116 mg |
| Opadry 85F2731 6 (Filmbildner) | 50 mg |

### Beispiel 2

Es wurden auf übliche Weise beschichtete Filmtabletten hergestellt, die pro Stück folgende

Bestandteile enthielten:

| | |
|---|---|
| Eisen(III)-Polymaltose-Komplex Fe(III)) | 226 mg (60 mg |
| Ascorbinsäure | 95 mg |
| Croscarmellose-Natrium | 41 mg |
| Kolloidales wasserfreies Siliziumoxid | 7 mg |
| Magnesiumstearat | 6 mg |
| Mikrokristalline Cellulose | 116 mg |
| Opadry 85F2731 6 (Filmbildner) | 50 mg |

### Beispiel 3

Bei der Untersuchung von Eisen(III)-Polymaltose-Komplex mit Ascorbinsäure (Ascorbinsäure) bei pH 3,0, 5,5 und 8,0 wurde folgendes gemessen:

| pH | Reaktionszeit [h] | Bildung von Fe²⁺-lonen [%] | Bildung von Dehydroascorbinsäure [%] |
|---|---|---|---|
| 3,0 | 2 | 2 | 2 |
| | 4 | 7 | 7 |
| 5,5 | 2 | 0 | 0 |
| 8,0 | 2 | 5 | 9 |
| | 4 | 5 | 13 |

Die gepufferten Lösungen (Puffer wie vorstehend beschrieben, s, Geisser, P., Arneim.-Forsch./Drug Res. 1990, 40 (II), 7, 754-760) wurden in einem molaren Verhältnis von Fe(III) ; Ascorbinsäure von 1 ;1 gemischt, wobei die Konzentration in der Mischung an Fe(III) und Ascorbinsäure je 5 x 10⁻⁵ mol/l betrug, und mit einem üblichen UV-VIS Spektrophotometer untersucht. Dabei wurde strikt unter Sauerstoffausschluss gearbeitet.

Aus der Tabelle ergibt sich klar, dass Eisen(III)-Polymaltose-Komplex mit Ascorbinsäure innerhalb von 4 Stunden bei pH-Werten zwischen 3 und 8 nur langsam zu Dehydroascorbinsäure und Fe(II) reagiert.

### Beispiel 4

### Klinische Studie

Es wurde die Wirkung von frisch gepresstem Orangensaft (Enhancer) und Tee (Inhibitor) auf die Aufnahme von markiertem ⁵⁹Fe in Erythrocyten nach der oralen Gabe von Eisen(III)-Polymaltose-Komplex bei Personen mit und ohne Eisenmangel untersucht.

### Methodologie:

Es handelt sich um eine Ein-Zentren-Kreuzstudie. Jede Testperson nahm an zwei Perioden teil, während derer eine Einzeldose von 100 mg Eisen als mit ⁵⁹Fe markierter Eisen(III)-Polymaltose-Komplex gegeben wurde (markiertes Maltofer^{®}(Vifor (International) AG, Schweiz)). Während einer Periode fasteten die Testpersonen vor der Gabe des Präparats über Nacht, während der anderen erhielten sie vor der Medikamentengabe definierte Nahrung (Gruppe A und Gruppe B). Als Alternative wurde die Medikation im gesättigten Zustand mit einem Eisenaufnahme-Enhancer (Orangensaft) oder einem Eisenaufnahme-Inhibitor (schwarzer Tee) gegeben (Gruppe C und Gruppe D). Insgesamt nahmen 32 Personen an der Studie teil. Es handelt sich sowohl um gesunde Personen als auch um solche mit Eisenmangel. Im Detail wurden die Gruppen wie folgt aufgeteilt:
Gruppe A: Personen mit Eisenmangel, die das Testmedikament aufeinanderfolgend jeweils im Zustand nach standardisierter Nahrungsaufnahme bzw. nach Fasten über Nacht erhielten.
Gruppe B: Normale Testpersonen, die das Testmedikament aufeinanderfolgend jeweils im Zustand nach standardisierter Nahrungsaufnahme bzw. nach Fasten über Nacht erhielten.
Gruppe C: Personen mit Eisenmangel, die das Testmedikament aufeinanderfolgend im Zustand nach standardisierter Nahrungsaufnahme gemeinsam jeweils mit Orangensaft bzw. mit schwarzem Tee erhielten.
Gruppe D: Normale Personen, die das Testmedikament aufeinanderfolgend im Zustand nach standardisierter Nahrungsaufnahme gemeinsam jeweils mit Orangensaft bzw. mit schwarzem Tee erhielten.

Das Testmedikament wurde in Gruppe A und B zusammen mit 100 ml Leitungswasser verabreicht, entweder auf nüchternen Magen (d.h. nach Fasten über Nacht) oder nach einem standardisierten Frühstück.

In Gruppe C und D wurde das Testmedikament nach einem standardisierten Frühstück mit 100 ml frisch gepresstem Orangensaft (entsprechend einem nach üblichen Methoden bestimmten Ascorbinsäure-Gehalt von 150 mg) oder mit 1 00 ml schwarzem Tee gegeben.

In allen Gruppen wurden für den Test jeweils 2 ml ⁵⁹Fe-markierte Maltofer®-Tropfen, entsprechend 100 mg Eisen, in jeweils 100 ml Wasser, Orangensaft bzw, schwarzen Tee (Earl Grey, 1 Teebeutel auf 100 ml Wasser, 4 min ziehen lassen) gegeben und damit zusammen eingenommen. Die verwendete Tasse wurde sofort mit 100 ml Wasser gespült und dieses Wasser ebenfalls getrunken.

Bei Gabe des Testmedikaments im Zustand nach Nahrungsaufnahme wurde das standardisierte Frühstück 30 Minuten vor der Gabe serviert und musste innerhalb von 30 Minuten beendet sein.

Zusätzlich erhielten alle Testpersonen standardisierte Mittagessen, Nachmittags-Snacks und Abendessen ca. 4, 6 bzw. 9 Stunden nach der Gabe des Testmedikaments.

Die Testpersonen erfüllten die folgenden Anforderungen:
- Hämoglobin < 130 g/l (Eisenmangel) oder ≥ 130 g/l (normal)
- Transferrin-Sättigung < 16 % oder Ferritin < 30 µg/l (Eisenmangel), Transferrinsättigung ≥ 16 % oder Ferritin ≥ 30 µg/l (normal).
- Keine weitere Ursache für Anämie (Thalassämie, maligne Tumore, chronische Infektionen usw.)

### Testprodukt

100 mg Eisen wurde oral als 2 ml ⁵⁹Fe-markierte Eisen(III)-Polymaltose-Komplex-Lösung (Maltofer^{®}Tropfen, Vifor (international) AG, Schweiz) in einer Konzentration von 50 mg elementarem Eisen/ml verabreicht, Es handelt sich um einen makromolekularen Komplex (Molekulargewicht 53.200 Dalton), Die Markierung des vom Hersteller bezogenen Testmedikaments Maltofer^{®} Tropfen erfolgte im GIN Laboratory, Uppsala University, Schweden nach einer eigenen Herstellungsvorschrift entsprechend GMP and GLP. Es wurden jeweils zwei einzelne Dosen gegeben, unterbrochen durch eine Ausscheidungs-Periode von mindestens 21 Tagen. Die verabreichte Radioaktivität in den beiden Perioden war:
Periode 1: 1 MBq ⁵⁹Fe
Periode 2: 2 MBq ⁵⁹Fe

### Pharmakokinetik und Effizienz

Die primäre Pharmakokinetik- und Effizienz-Variable war der ⁵⁹Fe-Einbau in Erythrocyten. Der sekundäre Endpunkt war die ⁵⁹Fe-Aktivität in Plasma, Proben zur Bestimmung von ⁵⁹Fe in Plasma und Erythrocyten wurden 96 Stunden nach Medikamentengabe und am Tag 7, 14 und 21 nach der Medikamentengabe entnommen. Die Proben wurden in EDTA-Röhrchen gegeben und innerhalb von 60 Minuten zentrifugiert, Proben von Plasma und Erythrocyten wurden bis zur Analyse gekühlt gelagert.

Es wurden folgende Parameter gemessen:
- Hämatologie: Blut-Hämoglobin, Blut-Hämatokrit, Blut-Leukozytenzahl, Blut-RBC, Blut-WBC, Erythrozyten-MCV, MCH, MCHC, Blutplättchen, Serum-TIBC, Serum-Fe, Serum-Transferrinsättigung, Serum-Ferritin
- Klinische Chemie: Serum-Cyanocobalamin (B₁₂), Serum-Folat, RBC-Folat

Ein Plateau in der Erythrozyten-Aufnahme-Kurve wurde bei ca. 20 Tagen/3 Wochen nach der Gabe des Testmedikaments erwartet. Das Blutvolumen wurde aus der Größe und dem Gewicht der Testpersonen nach Nadler et al, (Nadler, S.B., Surgery, 1962, 224-232) bestimmt, Die gemessene Blut-Radioaktivitätskonzentration wurde zur Ermittlung der Gesamtmenge an im Blut zirkulierenden ⁵⁹Fe mit dem Blutvolumen multipliziert. Dieser Wert wurde durch die Menge an verabreichtem ⁵⁹Fe dividiert, um den primären Effizienz-Endpunkt zu ermitteln, d,h, den eingebauten Prozentsatz der verabreichten Dosis.

Die Aufnahme-Werte nach 3 Wochen wurden dann für die statistische Auswertung verwendet. In der zweiten Behandlungsperiode wurden 2 MBq gegeben, und es konnte ein Restrauschen von der Gabe von 1 MBq während der ersten Studienperiode erwartet werden. Der 3-Wochen-Aufnahmewert nach Gabe von 2 MBq in der zweiten Periode wurde daher durch Subtraktion des 3-Wochen-Aufnahmewerts nach Gabe von 1 MBq in der ersten Periode korrigiert.

Weiterhin erfolgte ein Vergleich der Aufnahme von ⁵⁹Fe zwischen anämischen und normalen Testpersonen und die Beurteilung der ⁵⁹Fe-Aktivität im Plasma nach oraler Gabe von Eisen(II)-Polymaltose-Komplex durch deskriptive Plasma-Zeit-Aktivitöts-Profile.

Die Auswertung der Daten erfolgte mit üblichen statistischen Verfahren.

### Zusammenfassung der Ergebnisse

Ausgedrückt als relativen Einbau von Eisen in Erythrocyten, profitierten sowohl Testpersonen mit Eisenmangel als solche ohne Eisenmangel von der gleichzeitigen Gabe des Orangensaft-Enhancers mit Maltofer^{®}-Tropfen.

Der Vergleich der Eisenaufnahme in Erythrocyten zwischen anämischen und normalen Testpersonen erfolgte mit dem Student-Test im 5 % Niveau (einseitig).

Die Ergebnisse der Erythrocyten-Aufnahme nach der Behandlung mit Maltofer^{®}-Tropfen nach Fasten und nach Nahrungsaufnahme sind wie folgt:

**Tabelle 2:**

| | | | Eisenmangel | Kein Eisenmangel | |
|---|---|---|---|---|---|
| Parameter | | Nach Fasten | Nach Nahrungsaufnahme | nach Fasten | Nach Nahrungsaufnahme |
| ⁵⁹Fe Aufnahme in Erythrocyten (%) | N | 8 | 8 | 8 | 8 |
| | Mittel | 1,615 | 1,941 | 1,411 | 0,924 |
| | Geom. Mittel | 0,766 | 1,165 | 1,208 | 0,866 |
| | SD | 1,539 | 1,184 | 0,842 | 0,299 |
| | Median | 1,5 | 1,23 | 1,10 | 0,98 |
| | Min. | 0,06 | 0,24 | 0,51 | 0,33 |
| | Max. | 4,25 | 4,83 | 2,85 | 1,39 |

Die Ergebnisse der Erythrocyten-Aufnahme nach Gabe von Maltofer^{®}-Tropfen mit Orangensaft (Enhancer) oder schwarzem Tee (Inhibitor) sind wie folgt:

**Tabelle 3:**

| | | | Eisenmangel | Kein Eisenmangel | |
|---|---|---|---|---|---|
| Parameter | | Inhibitor | Enhancer | Inhibitor | Enhancer |
| ⁵⁹Fe Aufnahme in Erythrocyten (%) | N | 8 | 8 | 8 | 8 |
| | Mittel | 4,105 | 6,588 | 1,381 | 1,864 |
| | Geom. Mittel | 2,510 | 4,530 | 0,928 | 1,279 |
| | SD | 4,342 | 7,159 | 1,126 | 1,590 |
| | Median | 2,58 | 4,37 | 1,17 | 1,23 |
| | Min. | 0,33 | 1,26 | 0,25 | 0,33 |
| | Max. | 13,83 | 23,52 | 3,06 | 4,38 |

Die Punkt-Schätzung und das 90%-Konfidenzintervall des Verhältnisses der geometrischen Mittelwerte für den relativen Einbau von Eisen in Erythrocyten zwischen Zustand nach Fasten und nach Nahrungsaufnahem und zwischen Inhibitor und Enhancer (PP Set) sind wie folgt:

**Tabelle 4:**

| Zustand der Person | Verhältnis | Punkt-Schätzung | 90% Konfidenzintervall | |
|---|---|---|---|---|
| | | | Unteres | oberes |
| Eisenmangel | Fasten/Nahrung | 0,66 | 0,34 | 1,28 |
| Eisenmangel | Inhibitor/Enhancer | 0,55 | 0,36 | 0,86 |
| Kein Eisenmangel | Fasten/Nahrun | 1,39 | 0,97 | 1,99 |
| Kein Eisenmangel | Inhibitor/Enhancer | 0,73 | 0,41 | 1,28 |

Die Punkt-Schätzung, der p-Wert und das 90%-Konfidenzintervall des Verhältnisses des geometrischen Mittelwerts für den relativen Einbau von Eisen in Erythrocyten zwischen anämischen und normalen Personen nach Fasten, nach Nahrungsaufnahme, mit einem Inhibitor und mit einem Enhancer sind wie folgt:

**Tabelle 5:**

| Zustand der Person | Verhältnis | Punkt-Schätzung | p-Wert (einseitig) | 90% Konfidenzintervall | |
|---|---|---|---|---|---|
| | | | | unteres | oberes |
| Fasten | ID/ND¹ | 0,63 | 0,2324 | 0,22 | 1,81 |
| Nahrung | ID/ND¹ | 1,35 | 0,2551 | 0,63 | 2,88 |
| Mit Inhibitor | ID/ND¹ | 2,70 | 0,0440 | 1,04 | 7,03 |
| Mit Enhancer | ID/ND¹ | 3,54 | 0,0082 | 1,56 | 8,02 |

| | | | | | |
|---|---|---|---|---|---|
| ¹ ID = Eisenmangel; ND = kein Eisenmangel | | | | | |

Die in den obigen Tabellen 2 und 3 angegebenen Mittelwerte der Eisenaufnahme in Erythrocyten bei Personen mit Eisenmangel und solchen ohne Eisenmangel nach Fasten, nach Nahrungsaufnahme und mit Orangensaft sind in der folgenden Tabelle zusammengefasst,

**Tabelle 6:**

| Eisenmangel | | | | Kein Eisenmangel | | | |
|---|---|---|---|---|---|---|---|
| Orangensaft | Tee | Fasten | Nahrung | Orangensaft | Tee | Fasten | Nahrung |
| 6,58 | 4,105 | 1,615 | 1,941 | 1,84 | 1,381 | 1,411 | 0,924 |

Die Ergebnisse zeigen, dass die Eisenaufnahme (relativer Einbau von Eisen in Erythrocyten) bei Personen mit Eisenmangel verbessert wurde, wenn Maltofer^{®} mit Nahrung oder Orangensaft gegeben wurde, wobei der Effekt von Orangensaft auf die Eisenaufnahme deutlich höher ist als der von Nahrung. Ein Effekt des Inhibitors verglichen mit Nahrung ist nicht erkennbar. Auch normale Testpersonen profitierten von Orangensaft verglichen mit der Behandlung mit Maltofer^{®} zusammen mit einem Inhibitor, wobei der Effekt allerdings kleiner ist als bei Personen mit Eisenmangel, Bei normalen Testpersonen war die Eisenaufnahme nach Fasten größer als bei Gabe zusammen mit Nahrung, umgekehrt wie bei Personen mit Eisenmangel.

Es wurde weiter bestätigt, dass bei Personen mit Eisenmangel die Eisenaufnahme bei Gabe von Maltofer^{®} zusammen mit Nahrung, einem Enhancer oder Inhibitor größer war als bei normalen Personen. Eine größere Aufnahme wurde bei normalen Personen beobachtet, wenn Maltofer^{®} nach Fasten gegeben wurde.

Während der Studie zeigten sich keine schweren Nebenwirkungen von Maltofer^{®}-Tropfen. Die häufigsten Nebenwirkungen waren Kopfschmerzen, Diarrhoe und Bauchschmerzen in milder oder moderater Form; es wurden keine schweren Nebenwirkungen beobachtet.

## Patentansprüche

1. Präparat, umfassend einen oder mehrere Eisen(III)-Komplexverbindungen, die bei pH 7 ein Redoxpotential von -324 mV bis -750 mV gegenüber Normalwasserstoffelektrode (NHE) aufweisen, und eine oder mehrere redoxaktive Substanzen.

2. Präparat nach Anspruch 1 , wobei die redoxaktive(n) Substanz(en) ausgewählt ist/werden aus der aus Ascorbinsäure, Vitamin E, Cystein, physiologisch verträglichen Phenolen/Polyphenolen und Gluthathion bestehenden Gruppe, und insbesondere Ascorbinsäure ist,

3. Präparat nach Anspruch 1 oder 2, wobei die Eisen(III)-Komplexverbindungen Komplexverbindungen mit Kohlenhydraten oder Derivaten davon sind.

4. Präparat nach Anspruch 3, worin die Kohlenhydrate aus der Gruppe ausgewählt werden, die aus Dextranen und Derivaten davon, Dextrinen und Derivaten davon sowie Pullulan, Oligomeren und/oder Derivaten davon besteht.

5. Präparat nach den Anspruch 1, 2, 3 oder 4, worin die Kohlenhydrate aus oxidierten oder hydrierten Dextrinen ausgewählt werden.

6. Präparat nach Anspruch 1, 2, 3 oder 4, worin die Eisen(III)-Komplexverbindung eine Eisen(III)-Polymaltose-Komplexverbindung ist.

7. Präparat nach Anspruch 6, worin die Eisen(III)-Polymaltose-Komplexverbindung ein Molekulargewicht im Bereich von 20000 bis 500000 Dalton besitzt.

8. Präparat nach einem der Ansprüche 1 bis 7, worin die Eisen(III)-Komplexverbindung eine Eisen(III)-Komplexverbindung mit einem Oxidationsprodukt von einem oder mehreren Maltodextrinen ist.

9. Präparat nach Anspruch 8, worin die Eisen(III)-Komplexverbindung ein wasserlöslicher Eisen-Kohlenhydrat-Komplex ist, erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 37 und beim Einsatz eines Gemisches aus mehreren Maltodextrinen das Dextrose-Äquivalent des Gemisches bei 5 bis 37 und das Dextrose Äquivalent der am Gemisch beteiligten einzelnen Maltodextrine bei 2 bis 40 liegt,

10. Präparat nach einem der Ansprüche 1 bis 9 zur oralen Verabreichung.

11. Präparat nach einem der Ansprüche 1 bis 10 in Form von Tabletten, Granulat, Kapseln, Brausetabletten, Pulvermischung, Brausegranulat oder als Sachet.

12. Präparat nach einem der Ansprüche 1 bis 1 0 in der Form eines Kit aus einer Zubereitung, insbesondere einer Lösung, der Eisen(III)-Komplexverbindung und einer räumlich getrennt davon vorliegenden die redoxaktive(n) Substanz(en) enthaltenden Zubereitung.

13. Präparat nach Anspruch 12 in Form eines Single-Dose-Containers.

14. Präparat nach einem der Ansprüche 1 bis 1 2, darüber hinaus umfassend einen oder mehrere pharmakologisch wirksame Bestandteile, die ausgewählt werden aus der aus Vitaminen außer Ascorbinsäure, Spurenelementen, Cofaktoren, Mineralien und Nährstoffen bestehenden Gruppe.

15. Präparat nach einem der Ansprüche 1 2 bis 14, wobei der/die weiteren pharmakologisch wirksamen Bestandteil(e) in der Lösung des Eisen(III)-Komplexes oder in der Zubereitung der redoxaktiven Substanz(en), oder in beiden vorliegen können.

16. Präparat nach einem der Ansprüche 1 bis 15, wobei Eisen(III)-Komplexverbindung und Ascorbinsäure im Gewichtsverhältnis 1:0,05 bis 1 :20 vorliegen.

17. Präparat nach einem der Ansprüche 1 bis 1 6 als Medikament zur Behandlung von Eisenmangel-Zuständen.

18. Verwendung von Eisen(III)-Komplexen mit Kohlenhydraten oder Derivaten davon zur Herstellung eines Medikaments zur Behandlung von Eisenmangel-Zuständen, **dadurch gekennzeichnet, dass** das Medikament zeitnah oder gleichzeitig mit einer oder mehreren redoxaktiven Substanz(en) verabreicht wird.

19. Verwendung nach Anspruch 18, wobei das Medikament zur Behandlung von Eisenmangelzuständen bei Patienten mit chronisch entzündlicher Darmerkrankung, insbesondere Morbus Crohn oder Colitis ulcerosa, oder bei Schwangeren verwendet wird.

20. Verwendung von Eisen(III)-Komplexen mit Kohlenhydraten oder Derivaten davon zur Herstellung eines Medikaments zur Verbesserung der Immunabwehr, zur Steigerung der Hirnleistung und/oder zur Behandlung des "restless legs-Syndroms", **dadurch gekennzeichnet, dass** das Medikament zeitnah oder gleichzeitig mit einer oder mehreren redoxaktiven Substanz(en) verabreicht wird.

21. Verwendung nach Anspruch 18, wobei die redoxaktive(n) Substanz(en) in Lösung vorliegt/vorliegen, insbesondere in Form von Fruchtsaft.

22. Verwendung nach einem der Ansprüche 18 bis 21, wobei das Medikament darüber hinaus weitere pharmakologisch wirksame Bestandteile enthält, die ausgewählt werden aus der aus Vitaminen abgesehen von Ascorbinsäure, Spurenelementen, Cofaktoren, Mineralien und Nährstoffen bestehenden Gruppe.

## Geänderte Patentansprüche

### Geänderte Patentansprüche gemäss Regel 86(2) EPÜ.

**1.** Präparat, umfassend einen oder mehrere Eisen(III)-Komplexverbindungen, die bei pH 7 ein Redoxpotential von -324 mV bis -750 mV gegenüber Normalwasserstoffelektrode (NHE) aufweisen, und eine oder mehrere redoxaktive Substanzen, worin die Kohlenhydrate aus der Gruppe ausgewählt werden, die aus Dextranen und hydrierten Dextranen, Dextrinen, oxidierten oder hydrierten Dextrinen, sowie Pullulan, Oligomeren davon und/oder hydrierten Pullulanen besteht, und wobei die redoxaktive(n) Substanz(en) ausgewählt ist/werden aus der aus Ascorbinsäure, Vitamin E, Cystein, aus der aus Quercetin, Rutin, Flavonen, Flavonoiden, Hydrochinonen bestehenden Gruppe ausgewählten physiologisch verträglichen Phenolen/Polyphenolen, und Gluthathion bestehenden Gruppe, und insbesondere Ascorbinsäure ist.

**2.** Präparat nach Anspruch 1, worin die Eisen(III)-Komplexverbindung eine Eisen(III)-Polymaltose-Komplexverbindung ist.

**3.** Präparat nach Anspruch 2, worin die Eisen(III)-Polymaltose-Komplexverbindung ein Molekulargewicht im Bereich von 20.000 bis 500.000 Dalton besitzt.

**4.** Präparat nach einem der Ansprüche 1 bis 3, worin die Eisen(III)-Komplexverbindung eine Eisen(III)-Komplexverbindung mit einem Oxidationsprodukt von einem oder mehreren Maltodextrinen ist.

**5.** Präparat nach Anspruch 4 , worin die Eisen(III)-Komplexverbindung ein wasserlöslicher Eisen-Kohlenhydrat-Komplex ist, erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 37 und beim Einsatz eines Gemisches aus mehreren Maltodextrinen das Dextrose-Äquivalent des Gemisches bei 5 bis 37 und das Dextrose Äquivalent der am Gemisch beteiligten einzelnen Maltodextrine bei 2 bis 40 liegt.

**6.** Präparat nach einem der Ansprüche 1 bis 5 zur oralen Verabreichung.

**7.** Präparat nach einem der Ansprüche 1 bis 6 in Form von Tabletten, Granulat, Kapseln, Brausetabletten, Pulvermischung, Brausegranulat oder als Sachet.

**8.** Präparat nach einem der Ansprüche 1 bis 6 in der Form eines Kit aus einer Zubereitung, insbesondere einer Lösung, der Eisen(III)-Komplexverbindung und einer räumlich getrennt davon vorliegenden die redoxaktive(n) Substanz(en) enthaltenden Zubereitung.

**9.** Präparat nach Anspruch 8 in Form eines Single-Dose-Containers.

**10.** Präparat nach einem der Ansprüche 1 bis 9, darüber hinaus umfassend einen oder mehrere pharmakologisch wirksame Bestandteile, die ausgewählt werden aus der aus Vitaminen außer Ascorbinsäure, Spurenelementen, Cofaktoren, Mineralien und Nährstoffen bestehenden Gruppe.

**11.** Präparat nach einem der Ansprüche 8 bis 1 0, wobei der/die weiteren pharmakologisch wirksamen Bestandteil(e) in der Lösung des Eisen(III)-Komplexes oder in der Zubereitung der redoxaktiven Substanz(en), oder in beiden vorliegen können.

**12.** Präparat nach einem der Ansprüche 1 bis 11, wobei Eisen(III)-Komplexverbindung und Ascorbinsäure im Gewichtsverhältnis 1:0,05 bis 1:20 vorliegen.

**13.** Präparat nach einem der Ansprüche 1 bis 1 2 als Medikament zur Behandlung von Eisenmangel-Zuständen,

**14.** Verwendung von Eisen(III)-Komplexen mit Kohlenhydraten oder Derivaten davon wie in einem der Ansprüche 1 bis 5 definiert zur Herstellung eines Medikaments zur Behandlung von Eisenmangel-Zuständen, **dadurch gekennzeichnet, dass** das Medikament zeitnah oder gleichzeitig mit einer oder mehreren redoxaktiven Substanz(en) verabreicht wird, die wie in Anspruch 1 definiert ist/sind.

**15.** Verwendung nach Anspruch 14, wobei das Medikament zur Behandlung von Eisenmangelzuständen bei Patienten mit chronisch entzündlicher Darmerkrankung, insbesondere Morbus Crohn oder Colitis ulcerosa, oder bei Schwangeren verwendet wird.

**16.** Verwendung von Eisen(III)-Komplexen mit Kohlenhydraten oder Derivaten davon wie in einem oder mehreren der Ansprüche 1 bis 5 definiert zur Herstellung eines Medikaments zur Verbesserung der Immunabwehr, zur Steigerung der Hirnleistung und/oder zur Behandlung des "restless legs-Syndroms",
**dadurch gekennzeichnet, dass**
das Medikament zeitnah oder gleichzeitig mit einer oder mehreren redoxaktiven Substanz(en) verabreicht wird, die wie in Anspruch 1 definiert ist/sind.

**17.** Verwendung nach Anspruch 16, wobei die redoxaktive(n) Substanz(en) in Lösung vorliegt/vorliegen, insbesondere in Form von Fruchtsaft,

**18.** Verwendung nach einem der Ansprüche 14 bis 1 7, wobei das Medikament darüber hinaus weitere pharmakologisch wirksame Bestandteile enthält, die ausgewählt werden aus der aus Vitaminen abgesehen von Ascorbinsäure, Spurenelementen, Cofaktoren, Mineralien und Nährstoffen bestehenden Gruppe.
